# EUROPÄISCHE PATENTSCHRIFT

(11) **EP 0 246 549 B1**
(45) Veröffentlichungstag und Bekanntmachung des Hinweises auf die Patenterteilung: **02.02.1994**
(21) Anmeldenummer: 87106884.7
(22) Anmeldetag: 12.05.1987
(51) Int. Cl.: A61L 15/16

(54) **Transdermales Arzneimittel**
Transdermal medicament
Médicament transdermique

(30) Priorität: 22.05.1986 DE 3617158
(43) Veröffentlichungstag der Anmeldung: 25.11.1987
(73) Patentinhaber: LTS Lohmann Therapie-Systeme GmbH & Co. KG, 56513 Neuwied (DE)
(72) Erfinder: Guse, Günter, Dr., Dipl. Chem., D-2000 Hamburg 73 (DE); Asmussen, Bodo, Dr., D-2071 Ammersbek (DE); Borchert, Günter, D-2000 Hamburg 74 (DE)
(74) Vertreter: Flaccus, Rolf-Dieter, Dr.

(56) Entgegenhaltungen:
- EP-A- 0 181 970
- EP-A- 0 204 968
- GB-A- 2 100 605

## Beschreibung

Die Erfindung betrifft ein transdermales Arzneimittel, vorzugsweise in Form eines Pflasters, welches mindestens einen festen Wirk- oder Hilfsstoff oder mindestens einen Wirkstoff adsorbiert an einen festen Hilfsstoff als Suspension in einer viscosen, vorzugsweise klebenden Matrix enthält, sowie ein Verfahren zur Herstellung einer derartigen Suspension.

Transdermale Arzneimittel in Form von Pflastern sind an sich nicht neu, jedoch weisen die bisher bekannt gewordenen Ausführungsformen verschiedene Nachteile auf. Derartige transdermale Arzneimittel müssen im Prinzip die Funktionen Wirkstoffreservoir, Freisetzungssteuerung und Selbsthaftung auf der Haut erfüllen.

Aus Gründen einer einfacheren und wirtschaftlichen Herstellbarkeit sind dafür Systeme des sogenannten Matrixtyps besonders erwünscht, bei denen eine einzige Schicht ("Matrix") alle drei obengenannten Funktionen übernimmt.

Die Herstellung derartiger Systeme bereitet jedoch Schwierigkeiten, wenn mindestens ein fester Wirk- oder Hilfsstoff oder mindestens ein Wirkstoff adsorbiert an einen festen Hilfsstoff in Form einer Suspension in einer Matrix eingesetzt werden soll. Ein derartiges Vorgehen kann beispielsweise erforderlich sein, um die Kristallisation des Wirkstoffs in der Matrix zu verhindern, um den Wirkstoff besser handhabbar zu machen, wie z.B. im Falle des Glycerintrinitrats, oder wenn es sich um einen schwer löslichen Wirkstoff handelt (z.B. Moxonidin).

In solchen Fällen ist es bei den bisher bekanntgewordenen transdermalen Arzneimitteln nicht gelungen, pharmazeutische und klebtechnische Erfordernisse gleichermaßen befriedigend zu berücksichtigen. So steht die Korngröße der pharmazeutischen Wirk- oder Hilfsstoffe oft in einem Mißverhältnis zur Dicke der Matrix, mit der Folge, daß Wirkstoff- oder Hilfsstoff-Partikel über die hautseitige Oberfläche der Matrix hinausragen und deren Klebfähigkeit beeinträchtigen, womit zwangsläufig der sichere, vollflächige Hautkontakt und damit der geregelte Wirkstofftransport von der Matrix in die Haut gefährdet werden.

Zur Umgehung dieser Probleme schlägt die DE-OS 33 15 272 daher einen mehrschichtigen Aufbau mit getrennter Reservoir- und Haftklebeschicht vor, wobei in der Haftklebeschicht auf den Zusatz von (festen) Trägerstoffen verzichtet wird.

Ferner wird beobachtet, daß es bei Verwendung handelsüblicher pharmazeutischer Wirk- oder Hilfsstoffe zu Produktionstörungen kommt, beispielsweise dadurch, daß diese nach dem Einmischen in die Lösung des Matrixpolymeren sedimentieren und somit zu einer Ungleichverteilung des Arzneistoffes führen. Auch tritt beim flächigen Ausstreichen und anschließenden Trocknen arzneistoffhaltiger Polymermatrices bes. dann störende Blasenbildung auf, wenn die Wirkstoff- oder Hilfsstoffpartikel Lufteinschlüsse enthalten, die als Blasenkeime wirken.

Es ist bisher nicht befriedigend gelungen, diesen Schwierigkeiten, von denen einige beispielsweise auch in der EP-OS 13 606 beschrieben sind, dadurch zu entgehen, daß die Wirk- oder Hilfsstoffe vor dem Einmischen in die Matrix trocken zerkleinert wurden, entweder weil sie nicht mahlbar waren, beim Mahlen durch Temperatur- und Schereinwirkung Veränderungen erlitten, oder weil sie nach erfolgter Zerkleinerung Sekundäragglomerate bildeten, die nicht redispergierbar waren.

Aufgabe der Erfindung war die Entwicklung eines transdermalen Arzneimittels, welches die vorher beschriebenen Nachteile vermeidet, sowie die Entwicklung eines Verfahrens, mit dem feste pharmazeutische Wirk- oder Hilfsstoffe bzw. an feste Hilfsstoffe adsorbierte Wirkstoffe in einem einzigen Arbeitsgang zerkleinert, benetzt, entlüftet und in einer Klebstoffmatrix suspendiert werden können, ohne unerwünschte Veränderungen durch Temperatur- oder Scherwirkung zu erleiden.

Es wurde gefunden, daß ein derartiges transdermales Arzneimittel in überraschend einfacher Weise erhalten werden kann, wenn die Suspension durch Naßmahlung in Gegenwart einer mittelviscosen Lösung der Klebstoffmatrix (oder bestimmter Bestandteile davon) hergestellt und in an sich bekannter Weise weiterverarbeitet wird, sofern der Mahlprozeß unter geringer Temperatur- und Scherbelastung in einer Mühle mit frei beweglichen Mahlkörpern abläuft. Insbesondere hat sich die herkömmliche Kugelmühle als vorzüglich geeignetes Instrument erwiesen, d.h. ein liegender, langsam rotierender Zylinder mit frei beweglichem Kugelbett. Um Abrieb und Produktverunreinigung zu minimieren, werden für pharmazeutische Zwecke Mahltrommeln und -kugeln aus Hartporzellan bevorzugt. Die Drehzahl wird so bemessen, daß die reibende Rollbewegung der Kugeln (cascading) stark ausgeprägt ist; als Richtwert kann n = 10/√D̅i̅ gelten (n = Drehzahl in min ⁻¹; Di = Trommel-Innendurchmesser in m). Dabei ist der Energieeintrag in die Mühle so gering, daß das Mahlgut nur unwesentlich erwärmt wird (von Raumtemperatur auf etwa 30°C).

Bei der Herstellung von Arzneistoffsuspensionen für transdermale Arzneimittel ist es häufig erforderlich, den pharmazeutischen Wirk- oder Hilfsstoff in einem hochviscosen Polymersystem gleichmäßig zu verteilen. Es kann dann zweckmäßig sein, nicht die gesamte Polymerphase in der Kugelmühle vorzulegen, sondern nur bestimmte Anteile davon, so daß während des Mahlprozesses eine mittlere Viscosität der fluiden Phase vorherrscht (vorzugsweise 100 bis 300, insbesondere 200 mPa s). Würde die Naßmahlung ausschließlich in Gegenwart einer niederviscosen Flüssigkeit, also etwa eines organischen Lösungsmittels, ablaufen, so erhielte man unerwünscht hohen Abrieb von Trommel und Kugeln.

Für die bevorzugte Ausführungsform der Herstellung eines Nitropflasters mit dem Wirkstoff Glycerintrinitrat, der hier in beispielhafter und nicht einschränkender Weise genannt wird, ergibt sich eine zweckmäßige und besonders bevorzugte Aufteilung der Polymerkomponenten aus Beispiel 1.

Bei dem dort beschriebenen Prozeß gelingt es, die mittlere Korngröße des eingesetzten Milchzuckers (von anfänglich 80 My m) in der Kugelmühle binnen 16 Stunden auf 20 My m herabzusetzen. Gleichzeitig wird die Sedimentationsneigung der fertigen Zubereitung stark vermindert und die Entstehung harter, nicht mehr redispergierbarer Bodensätze, wie sie bei Verfahren nach dem Stand der Technik möglich ist, gänzlich ausgeschlossen.

Sowohl die Mahlwirkung als auch die Verhinderung der Sedimentation können bei Bedarf in einer Ausführungsform der Erfindung noch dadurch begünstigt werden, daß man dem Kugelmühlenansatz kleine Mengen grenzflächenaktiver Stoffe zusetzt. In Betracht kommen beispielsweise Fettalkoholethoxilate, Sorbitanesterethoxilate und sulfatierte Ricinusöle in Mengen von etwa 0,2 bis 1 Gew.-% des Ansatzes.

Wünscht man einen besonders schnell ablaufenden Prozeß der Suspensionsbildung, so kann man gemäß einer anderen Ausführungsform der Erfindung bei weniger temperatur- und scherempfindlichen Systemen auch Mühlen mit höherem Energieeintrag verwenden wie beispielswiese die Rührwerkskugelmühle (Perlmühle), bei der eine Vorsuspension durch einen stehenden Zylinder gepumpt wird, in dem ein hochtouriges Rührorgan ein Kugelbett umwälzt.

Bei der beschriebenen Herstellung einer Suspension als Teilkomponente einer Nitropflaster-Streichmasse ist dagegen die Herstellung einer Vorsuspension entbehrlich. Dieser Prozeß gemäß der bevorzugten Ausführungsform der Erfindung hat gleichzeitig noch einen gewichtigen Vorteil. Die vorzugsweise eingesetzte handelsübliche Verreibung von 10 % Glycerintrinitrat und 90 % Milchzucker fällt nach den in der Bundesrepublik Deutschland geltenden Vorschriften unter die Gefahrgruppe B der Unfallverhütungsvorschrift 41 der Berufsgenossenschaft der Chemischen Industrie (UVV 41) und unterliegt damit erhöhten Arbeitssicherheitsanforderungen. Die fertige Kugelmühlensuspension gehört dagegen zu den vereinfacht handhabbaren Stoffen der Gefahrgruppe C, und zwar auch dann noch, wenn sie völlig von Lösungsmitteln befreit ist. Somit wird in der Kugelmühle, die leicht inertisiert und fest verschlossen werden kann, unter schonenden Bedingungen, die sowohl für die Produkt- wie für die Arbeitssicherheit besonders günstig sind, eine handhabungssichere Glycerintrinitrat-Zubereitung erzeugt. Die Handhabungssicherheit kann bei Bedarf noch weiter dadurch erhöht werden, daß man einen Teil des Lösungsmittels (normalerweise n-Heptan) durch Toluol, Aceton oder Essigsäureethylester ersetzt.

Streichmassen für transdermale Arzneimitteln des Matrixtyps, die unter Verwendung der erfindungsgemäßen Suspensionen hergestellt sind, können in an sich bekannter Weise auf flächige Trägermaterialien ausgestrichen und darauf getrocknet werden. Sie zeigen beim Trocknen, verglichen mit Streichmassen nach dem Stand der Technik, eine erheblich verminderte Tendenz zur Blasenbildung.

Die Klebeigenschaften, d.h. die subjektiv empfundene Anfaßklebrigkeit (Soforthaftung, "Tack") und die Verklebungsfestigkeit auf der Haut während der üblichen Tragezeit von 24 Stunden oder mehr sind bei erfindungsgemäß hergestellten Pflastern deutlich besser als bei solchen nach dem Stand der Technik. Damit erfüllen die transdermalen Arzneimittel gemäß der vorliegenden Erfindung eine wesentliche Voraussetzung für den gleichmäßigen kontrollierten Wirkstofftransport aus der Pflastermatrix in die Haut.

Die Erfindung wird durch die nachstehenden Beispiele noch weiter erläutert, ohne sie auf die darin genannten Ausführungsformen zu beschränken. Die Abkürzung GT bedeutet Gewichtsteile.

### Beispiel 1

In eine Hartporzellan-Kugelmühle werden folgende Stoffe nacheinander eingefüllt:
39,86 GT 60 %ige Lösung von Polyisobuten Mᵥ = 40.000 in n-Heptan
91,95 GT n - Heptan
6,43 GT 5 %ige Lösung von Glycerintrinitrat in Neutralöl
1,03 GT sulfatiertes Ricinusöl
37,57 GT aliphatisches Kohlenwasserstoffharz, Erweichungspunkt R + K 97°C
Die Mühle wird verschlossen und rotiert 1 Stunde, um den Inhalt zu homogenisieren. Sodann werden eingefüllt:
85,36 GT 10%ige Verreibung von Glycerintrinitrat auf Lactose
Die erneut verschlossene Mühle rotiert anschließend etwa 20 Stunden. Danach ist eine homogene gelbliche Suspension mit einer Viscosität von etwa 250 mPa s entstanden. Durch Grindometermessung wird gefunden, daß die Korngröße der Lactose von anfänglich 80 My m auf max. 25 My m herabgesetzt worden ist.

Die Suspension wird mit einer homogenen, viscosen, im Kneter hergestellten Lösung aus
41,37 GT Polyisobuten Mᵥ = 2.800.000
33,95 GT 60 %ige Lösung von Polyisobuten Mᵥ = 40.000 in n-Heptan
177,55 GT n-Heptan
durch Rühren vermischt. Dieses Gemisch ist eine streichfertige Selbstklebemasse, die in an sich bekannter Weise zur Herstellung eines selbstklebenden Pflaster mit Glycerintrinitrat verwendet werden kann.

### Beispiel 2

Ein Copolymerisat aus
65 Gew.-% 2-Ethylhexylacrylat
15 Gew.-% n-Butylacrylat
20 Gew.-% N-Vinylpyrrolidon
wird durch Lösungspolymerisation in Benzin/Aceton hergestellt (K-Wert 74,3).

249,4 g dieser Polymerisatlösung, deren Festkörpergehalt 39,7 % beträgt, werden mit 1,0 g Moxonidin in einer Hartporzellan-Kugelmühle 24 Stunden lang vermahlen.

Es resultiert eine homogene, weitgehend sedimentationsfreie, streichfertige Wirkstoffsuspension.

## Patentansprüche (Patentansprüche für folgende(n) Vertragsstaat(en): AT, BE, CH, DE, FR, GB, IT, LI, NL, SE)

1. Verfahren zur Herstellung eines transdermalen Arzneimittels, welches mindestens einen festen Wirk- oder Hilfsstoff oder mindestens einen Wirkstoff, adsorbiert an einen festen Hilfsstoff, als Suspension in einer viskosen Matrix enthält, dadurch gekennzeichnet, daß die Herstellung der Suspensionen durch Naßmahlung in Gegenwart einer mittelviskosen Lösung der Matrix oder bestimmter Bestandteile davon unter geringer Temperatur und Scherbelastung erfolgt, gegebenenfalls unter Zusatz geringer Mengen grenzflächenaktiver Stoffe beim Mahlen.

2. Verfahren nach Anspruch 1, dadurch gekennzeichnet, daß die Mahlung in einer Mühle mit frei beweglichen Mahlkörpern oder in einer Kugelmühle durchgeführt wird.

3. Transdermales Arzneimittel, welches mindestens einen festen Wirk- oder Hilfsstoff oder mindestens einen Wirkstoff, adsorbiert an einen festen Hilfsstoff, als Suspension in einer viskosen Matrix enthält, dadurch gekennzeichnet, daß es erhältlich ist durch ein Verfahren nach Anspruch 1 oder 2.

4. Transdermales Arzneimittel nach Anspruch 3, dadurch gekennzeichnet, daß es sich um ein Pflaster handelt.

5. Transdermales Arzneimittel nach Anspruch 3, dadurch gekennzeichnet, daß es sich bei der Matrix um eine Klebstoffmatrix handelt.

6. Transdermales Arzneimittel nach Anspruch 3, dadurch gekennzeichnet, daß es die Suspension in einer Streichmasse auf einem flächigen Trägermaterial enthält.

7. Transdermales Arzneimittel nach Anspruch 3, dadurch gekennzeichnet, daß es sich bei dem Wirk- und Hilfsstoff um Glycerintrinitrat und Lactose handelt.

8. Transdermales Arzneimittel nach Anspruch 3, dadurch gekennzeichnet, daß es sich bei dem festen Wirkstoff um Moxonidin handelt.

## Patentansprüche (Patentansprüche für folgende(n) Vertragsstaat(en): ES)

1. Verfahren zur Herstellung eines transdermalen Arzneimittels, welches mindestens einen festen Wirk- oder Hilfsstoff oder mindestens einen Wirkstoff, adsorbiert an einen festen Hilfsstoff, als Suspension in einer viskosen Matrix enthält, dadurch gekennzeichnet, daß die Herstellung der Suspensionen durch Naßmahlung in Gegenwart einer mittelviskosen Lösung der Matrix oder bestimmter Bestandteile davon unter geringer Temperatur und Scherbelastung erfolgt, gegebenenfalls unter Zusatz geringer Mengen grenzflächenaktiver Stoffe beim Mahlen.

2. Verfahren nach Anspruch 1, dadurch gekennzeichnet, daß die Mahlung in einer Mühle mit frei beweglichen Mahlkörpern oder in einer Kugelmühle durchgeführt wird.

## Claims (Claims for the following Contracting State(s): AT, BE, CH, DE, FR, GB, IT, LI, NL, SE)

1. Process for the production of a transdermal medicament, containing at least one solid active substance or adjuvant, or at least one active substance adsorbed to a solid adjuvant, as a suspension in a viscous matrix, characterized in that the preparation of the suspensions is carried out by wet milling in the presence of a medium-viscous solution of the matrix or particular components thereof at low temperature and shear stress, optionally adding small amounts of surface-active substances during milling.

2. Process according to claim 1, characterized in that the milling is carried out in a mill with freely moving milling bodies or in a ball mill.

3. Transdermal medicament containing at least one solid active substance or adjuvant or at least one active substance adsorbed to a solid adjuvant, as a suspension in a viscous matrix, characterized in that it can be prepared employing the process according to claim 1 or 2.

4. Transdermal medicament according to claim 3, characterized in that it is a plaster.

5. Transdermal medicament according to claim 3, characterized in that the matrix is an adhesive matrix.

6. Transdermal medicament according to claim 3, characterized in that it contains the suspension in a coating mass on a sheet-like carrier material.

7. Transdermal medicament according to claim 3, characterized in that the active substance and adjuvant are glyceryl nitrate and lactose, respectively.

8. Transdermal medicament according to claim 3, characterized in that the solid active substance is moxonidine.

## Claims (Claims for the following Contracting State(s): ES)

1. Process for the production of a transdermal medicament, containing at least one solid active substance or adjuvant, or at least one active substance adsorbed to a solid adjuvant, as a suspension in a viscous matrix, characterized in that the preparation of the suspensions is carried out by wet milling in the presence of a medium-viscous solution of the matrix or particular components thereof at low temperature and shear stress, optionally adding small amounts of surface-active substances during milling.

2. Process according to claim 1, characterized in that the milling is carried out in a mill with freely moving milling bodies or in a ball mill.

## Revendications (Revendications pour l'(les) Etat(s) contractant(s) suivant(s): AT, BE, CH, DE, FR, GB, IT, LI, NL, SE)

1. Procédé de préparation d'un médicament transdermique qui contient au moins une substance active ou adjuvante solide, ou au moins une substance active, adsorbée sur une substance active solide, sous forme de suspension, dans une matrice visqueuse, caractérisé en ce que la préparation des suspensions s'effectue par broyage à l'état mouillé en présence d'une solution de viscosité moyenne de la matrice ou de constituants déterminés de celle-ci, sous faible charge de température et de cisaillement, en recourant éventuellement à l'addition, lors du broyage, de faibles quantités de substances tensioactives.

2. Procédé suivant la revendication 1, caractérisé en ce que l'on entreprend le broyage dans un broyeur avec des corps de broyage librement mobiles ou dans un broyeur à boulets.

3. Médicament transdermique, qui contient au moins une substance active ou adjuvante solide, ou au moins une substance active, adsorbée sur une substance adjuvante solide, sous forme de suspension, dans une matrice visqueuse, caractérisé en ce qu'il est obtenu par mise en oeuvre du procédé suivant l'une quelconque des revendications 1 et 2.

4. Médicament transdermique suivant la revendication 3, caractérisé en ce qu'il s'agit d'un emplâtre.

5. Médicament transdermique suivant la revendication 3, caractérisé en ce qu'il s'agit, dans le cas de la matrice, d'une matrice de colle.

6. Médicament transdermique suivant la revendication 3, caractérisé en ce qu'il contient la suspension dans une masse étalée sur un matériau de support plat.

7. Médicament transdermique suivant la revendication 3, caractérisé en ce que, dans le cas de la substance active et de la substance adjuvante, il s'agit de trinitrate de glycérine et de lactose.

8. Médicament transdermique suivant la revendication 3, caractérisé en ce que, dans le cas de la substance active solide, il s'agit de moxonidine.

## Revendications (Revendications pour l'(les) Etat(s) contractant(s) suivant(s): ES)

1. Procédé de préparation d'un médicament transdermique qui contient au moins une substance active ou adjuvante solide, ou au moins une substance active, adsorbée sur une substance active solide, sous forme de suspension, dans une matrice visqueuse, caractérisé en ce que la préparation des suspensions s'effectue par broyage à l'état mouillé en présence d'une solution de viscosité moyenne de la matrice ou de constituants déterminés de celle-ci, sous faible charge de température et de cisaillement, en recourant éventuellement à l'addition, lors du broyage, de faibles quantités de substances tensioactives.

2. Procédé suivant la revendication 1, caractérisé en ce que l'on entreprend le broyage dans un broyeur avec des corps de broyage librement mobiles ou dans un broyeur à boulets.
